# EUROPEAN PATENT APPLICATION

(11) **EP 1 862 071 A2**
(43) Date of publication of application: **05.12.2007**
(21) Application number: 07109427.0
(22) Date of filing: 01.06.2007
(51) Int. Cl.: A01N 65/00, A01N 57/12, A01N 31/02, A01P 1/00

(54) **Sterilization solution and method for manufacturing sterilization solution**

(30) Priority: 02.06.2006 JP 2006154236
(71) Applicant: Dream Do Co., Ltd., Tokyo 104-0032 (JP)
(72) Inventor: Miyake, Kazuyuki, Chiba-ken 260-0821 (JP)
(74) Representative: Viering, Jentschura & Partner

(57) **Abstract**

An inactivation effect of GSE on Norovirus is revealed and a. sterilization solution suitable for inactivating Norovirus is provided. The sterilization solution has an effect of inactivating Calicivirus, and is prepared by dissolving a grapefruit seed extract (hereafter abbreviated as GSE) into water, adding phytic acid, and adding brewer's alcohol. In the sterilization solution, GSE is 0.08 percent by weight, phytic acid is 0.05 percent by weight, brewer's alcohol is 58.8 percent by weight, and water is the remainder. The pH is adjusted to become 5.8 to 6.4. A method for manufacturing a sterilization solution, having an effect of inactivating Calicivirus includes the steps of dissolving GSE into water, adding phytic acid so as to emulsify, and adding 95-degree brewer's alcohol.

## Description

### BACKGROUND OF THE INVENTION

### 1. Field of the Invention

The present invention relates to a sterilization solution.

### 2. Description of the Related Art

International Patent Publication WO 98/12924 discloses a sterilization solution primarily containing an organic acid aqueous solution containing pure copper ions, prepared by mixing a fine powder of copper into an aqueous solution in which pH is controlled by using the organic acid.

Japanese Unexamined Patent Application Publication No. 2000-355506 discloses a sterilization solution prepared by mixing alcohol, a humectant, and water.

On the other hand, a grapefruit seed extract (GSE) is a natural food additive. Since the sterilization effect thereof was ascertained in 1976, effects of inactivating many gram-positive bacteria, gram-negative bacteria, mold genera, e.g., Escherichia coli 0157, Salmonella, Vibrio parahemolyticus, and Staphylococcus aureus, have been made clear.

However, an effect exerted on a virus is not certain.

### SUMMARY OF THE INVENTION

The present invention reveals an inactivation effect of GSE on Norovirus and provides a sterilization solution suitable for inactivating Norovirus.

A sterilization solution according to an aspect of the present invention has an effect of inactivating Calicivirus and is prepared by dissolving GSE into water, adding phytic acid, and adding brewer's alcohol. In the sterilization solution, GSE may be 0.07 to 0.09 percent by weight, phytic acid may be 0.04 to 0.06 percent by weight, brewer's alcohol may be 58 to 59.9 percent by weight, and water may be the remainder. Preferably, GSE is 0.08 percent by weight, phytic acid is 0.05 percent by weight, brewer's alcohol is 58.8 percent by weight, and water is the remainder. It is desirable that the pH is adjusted to be 5.8 to 6.4. A method for manufacturing a sterilization solution according to an aspect of the present invention is a method for manufacturing a sterilization solution having an effect of inactivating Calicivirus and the method includes the steps of dissolving GSE into water, adding phytic acid so as to emulsify, and adding 95-degree brewer's alcohol. An adjustment may be performed in such a way that GSE becomes 0.07 to 0.09 percent by weight, phytic acid becomes 0.04 to 0.06 percent by weight, brewer's alcohol becomes 58 to 59.9 percent by weight, and water becomes the remainder. In particular, preferably, an adjustment is performed in such a way that GSE becomes 0.08 percent by weight, phytic acid becomes 0.05 percent by weight, brewer's alcohol becomes 58.8 percent by weight, and water becomes the remainder. Furthermore, preferably, an adjustment is performed in such a way that the pH becomes 5.8 to 6.4.

An effect of inactivating Feline Calicivirus (FCV), which is believed to be a virus closely related to Norovirus on the basis of morphological features and the structure of genome, is exerted. That is, an antibacterial effect of destructing Feline Calicivirus in a short time and maintaining the state, in which no virus is present, for a long time is exerted. Consequently, it is indicated that an effect of inactivating Norovirus will be exerted.

### BRIEF DESCRIPTION OF THE DRAWINGS

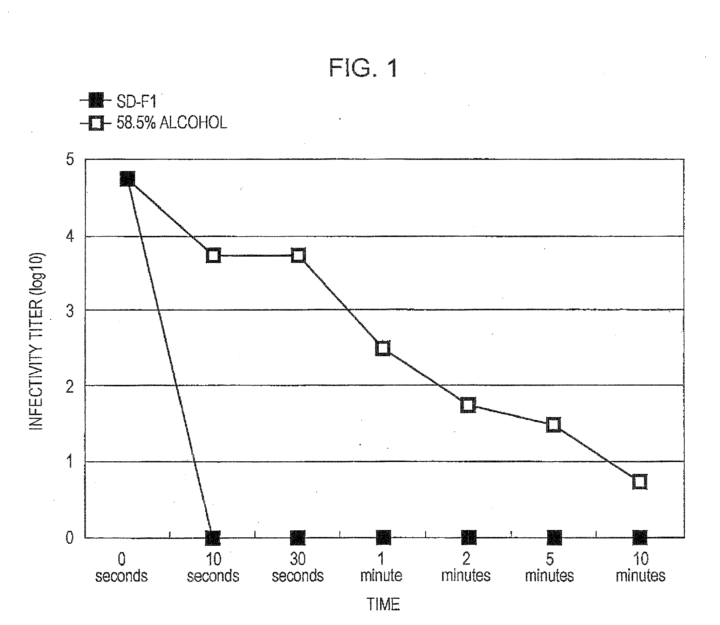

Fig. 1 is a graph showing Feline Calicivirus inactivation test results.

### DESCRIPTION OF THE PREFERRED EMBODIMENTS

Dream-Do Co., Ltd., to which the inventor of the present invention belongs, has developed a product, "Super Dream F-1" as a sterilization solution. Hereafter, Super Dream F-1 (" Super Dream F-1" is a trademark of Dream-Do Co., Ltd.) is referred to as "SD-F1", when necessary. The effects of "SD-F1" on coliforms and the like have been known from the beginning. Recently, in order to check an effect on Norovirus, a test was requested to Osaka Prefectural Institute of Public Health. As a result, an effect of inactivating Feline Calicivirus, which is an alternative virus to Norovirus, has been reported. The present invention has been made on the basis of the new findings.

3D-FI is a sterilization solution prepared by dissolving GSE into water, adding phytic acid, and adding brewer's alcohol. In SD-F1, GSE is 0.07 to 0.09 percent by weight, phytic acid is 0.04 to 0.06 percent by weight, brewer's alcohol is 58 to 59.9 percent by weight, and water is the remainder. In particular, an adjustment is performed in such a way that GSE becomes 0.08 percent by weight, phytic acid becomes 0.05 percent by weight, brewer's alcohol becomes 58.8 percent by weight, and water becomes the remainder. It is desirable that the pH is adjusted to be 5.8 to 6.4. A method for manufacturing SD-F1 includes the steps of dissolving GSE into water, adding phytic acid so as to emulsify, and adding 95-degree brewer's alcohol. An adjustment is performed in such a way that GSE becomes 0.07 to 0.09 percent by weight, phytic acid becomes 0.04 to 0.06 percent by weight, brewer's alcohol becomes 58 to 59.9 percent by weight, and water becomes the remainder. In particular, preferably, an adjustment is performed in such a way that GSE becomes 0.08 percent by weight, phytic acid becomes 0.05 percent by weight, brewer's alcohol becomes 58.8 percent by weight, and water becomes the remainder. Furthermore, an adjustment is performed in such a way that the pH becomes 5.8 to 6.4.

In order to check the inactivation effect of this SD-F1 on Norovirus, Feline Calicivirus (FCV); which is an alternative virus to Norovirus, is used. Since Norovirus itself has not been cultured stably, the experiment was carried out on Feline Calicivirus closely related to Norovirus on the basis of morphological features and the structure of genome. An FCV-F9 strain purchased from ATCC was used as Feline Calicivirus. For the growth of the virus and the measurement of the infectivity titer, the feline kidney (CRFK) cell purchased from Dainippon Pharmaceutical Co., Ltd., was used. For test specimens, SD-F1 (0.08% of GSE, 58.8% of brewer's alcohol, 0.05% of phytic acid) and 58.5% brewer's alcohol were used, and for reference, purified water were used (merely 0 minutes). In the inactivation experiment, each test specimen was blended with FCV, the amount of which was one-tenth the test specimen, and a sample was taken at room temperature after a predetermined time. The resulting sample was diluted immediately by a factor of 50 and, thereafter, the virus infectivity titer (TCID₅₀) was measured by a microplate method.

### Results and Discussion

SD-F1. wholly inactivated FCV exhibiting 5 × 10⁴ TCID₅₀ in 10 seconds. SD-F1 exerted the virus inactivation effect comparable to ,the effect of a medium or high sterilization disinfectant, e.g., 3% glutaral or 100 mg/L sodium hypochlorite (the 26th Annual Meeting of Japanese Society of Food Microbiology). Two minutes were required for 58.5% brewer's alcohol alone to inactivate by 99.9%. One minute was required for 80% rubbing alcohol (the 26th Annual Meeting of Japanese Society of Food Microbiology). It is believed that the antibacterial effect of GSE is due to flavonoid or aliphatic acid. It was observed with an electron microscope that cell walls of bacteria were dissolved by GSE and protoplasm was eluted within 15 minutes. Since a high inactivation effect on FCV was ascertained, it was indicated that SD-F1 was useful for prevention of food poisoning due to Norovirus.

**Table 1**

| | 0 seconds | 10 seconds | 30 seconds | 1 minute | 2 minutes | 5 minutes | 10 minutes |
|---|---|---|---|---|---|---|---|
| SD-F1 | 4.75 | 0 | 0 | 0 | 0 | 0 | 0 |
| 58.5% alcohol | 4.75 | 3.75 | 3.75 | 2.5 | 1.75 | 1.5 | 0.75 |

| | | | | | | | |
|---|---|---|---|---|---|---|---|
| A numeric value indicates log₁₀X, where X represents TCID₅₀ | | | | | | | |

**Table 2**

| | 0 seconds | 10 seconds | 30 seconds | 1 minute | 2 minutes | 5 minutes | 10 minutes |
|---|---|---|---|---|---|---|---|
| SD-F1 | 56000 | 0 | 0 | 0 | 0 | 0 | 0 |
| 58.5% alcohol | 56000 | 5600 | 5600 | 3200 | 56 | 32 | 6 |

| | | | | | | | |
|---|---|---|---|---|---|---|---|
| A numeric-value indicates TCID₅₀ | | | | | | | |

Table 1 shows the inactivation effects of SD-F1 and 58.5% brewer's alcohol on Feline Calicivirus. A numerical value indicates logarithm of TCID₅₀. In Table 2, a numerical value is not a logarithmic value but indicates TCID₅₀ itself. Fig. 1 is a graph of the results shown in Table 1.

A test result of the present invention as reported by Osaka Prefectural Institute of Public Health is now described. The test was carried out on sample name "Super Dream F-1" for the purpose of Feline Calicivirus Inactivation. The virus used was ATCC Feline Calicivirus F9 strain (FCV). The cell used was Feline kidney (CRFK) cell (Dainippon Pharmaceutical Co., Ltd.). The requested specimen was Super Dream F-1 solution 500 mL, for reference, 58.5% brewer's alcohol and purified water were used.

FCV was adjusted to exhibit about 10⁶ TCID₅₀ relative to 50 µL, 0.5 mL of the resulting FCV was blended with 4.5 mL of the test specimen, and 0.1 mL was taken at room temperature after each of 10 and 30 seconds, 1, 2, 5, and 10 minutes. Dilution was performed by a factor of 50 with 4.9 mL of Eagle MEM solution and, thereby, the action of the reagent was stopped. This was taken as a stock solution for measurement of the infectivity titer. For reference (0 seconds), purified water was used.

The measurement of the infectivity titer was performed by a micro method. The culture medium (10% fetal calf serum-containing Eagle MEM) of the microplate, on which CRFK cells were grown, was discarded, and 50 µL of virus reaction solution, which had been subjected to serial dilution to 10 ⁶ by a factor of 10, was put into four wells of the plate for each dilution. Adsorption was performed at 37°C for 1 hour. Thereafter, a maintenance medium (2% fetal calf serum-containing Eagle MEM) was added, and standing was performed at 37°C. After 5 days, the virus infectivity titer was determined where an occurrence of cell degeneration was taken as an indicator.

As a result, Super Dream F-1 wholly inactivated FCV exhibiting 5.6 × 10⁴ TCID₅₀ in 10 seconds. Two minutes were required for 58.5% alcohol to inactivate FCV by 99.9%.

### First embodiment

The sterilization solution according to an aspect of the present invention may be produced on one ton basis, for example. Immediately after the production, the sterilization solution may be stored into an easy-to-use volume of spray bottle (a bottle provided with a sprayer to be used for spraying liquid), and may be used in various instances where sterilization is required.

The sterilization solution according to an aspect of the present invention may be stored into an easy-to-use volume of spray bottle and may be used in various instances. For example, it may be used for general production lines, machinery and appliances, e.g., food processors, cooking tools, e.g., kitchen knives and chopping boards, and deodorization of hands and fingers after washing and garbage.

## Claims

1. A sterilization solution having an effect of inactivating Calicivirus, wherein the sterilization solution is prepared by dissolving a grapefruit seed extract (GSE) into water, adding phytic acid, and adding brewer's alcohol.

2. The sterilization solution according to Claim 1, wherein GSE is 0.07 to 0.09 percent by weight, phytic acid is 0.04 to 0.06 percent by weight, brewer's alcohol is 58 to 59.9 percent by weight, and water is the remainder.

3. The sterilization solution according to Claim 1, wherein GSE is 0.08 percent by weight, phytic acid is 0.05 percent by weight, brewer's alcohol is 58.8 percent by weight, and water is the remainder.

4. The sterilization solution according to Claim 1, wherein the pH is 5.8 to 6.4.

5. A method for manufacturing a sterilization solution having an effect of inactivating Calicivirus, the method comprising the steps of:
dissolving GSE into water;
adding phytic acid so as to emulsify; and
adding 95-degree brewer's alcohol.

6. The method for manufacturing a sterilization solution according to Claim 5, wherein an adjustment is performed in such a way that GSE becomes 0.07 to 0.09 percent by weight, phytic acid becomes 0.04 to 0.06 percent by weight, brewer's alcohol becomes 58 to 59.9 percent by weight, and water becomes the remainder.

7. The method for manufacturing a sterilization solution according to Claim 5, wherein an adjustment is performed in such a way that GSE becomes 0.08 percent by weight, phytic acid becomes 0.05 percent by weight, brewer's alcohol becomes 58.8 percent by weight, and water becomes the remainder.

8. The method for manufacturing a sterilization solution according to Claim 5, wherein an adjustment is performed in such a way that the pH becomes 5.8 to 6.4.
